# EUROPEAN PATENT SPECIFICATION

(11) **EP 2 223 919 B1**
(45) Date of publication and mention of the grant of the patent: **15.10.2014**
(21) Application number: 08864650.0
(22) Date of filing: 24.12.2008
(51) Int. Cl.: C07D 401/04, A01N 43/42, A01P 3/00, C07D 495/04

(54) **NITROGEN-CONTAINING HETEROCYCLIC COMPOUNDS AND THEIR USE AS AGRICULTURAL AND HORTICULTURAL FUNGICIDES**
STICKSTOFFHALTIGE HETEROCYCLISCHE VERBINDUNGEN UND IHRE VERWENDUNG ALS LANDWIRTSCHAFTLICHE UND GÄRTNERISCHE FUNGIZIDE
COMPOSÉS HÉTÉROCYCLIQUES CONTENANT DE L'AZOTE ET LEUR UTILISATION COMME FONGICIDES EN AGRICULTURE ET HORTICULTURE

(30) Priority: 26.12.2007 JP 2007334454; 14.02.2008 JP 2008032945
(43) Date of publication of application: 01.09.2010
(73) Proprietor: Nippon Soda Co., Ltd., Tokyo 100-8165 (JP)
(72) Inventor: MITANI, Akira, Odawara-shi Kanagawa 250-0280 (JP); KUWAHARA, Raito, Odawara-shi Kanagawa 250-0280 (JP)
(74) Representative: Wibbelmann, Jobst
(86) International application number: PCT/JP2008/003925
(87) International publication number: WO 2009/081579

(56) References cited:
- WO-A1-2005/070917
- WO-A1-2007/011022
- WO-A1-2008/066148
- JP-A- 62 294 679
- JP-A- 2007 001 944
- JP-A- 2007 217 353
- JP-A- 2007 269 686
- WERMUTH C G: "MOLECULAR VARIATIONS BASED ON ISOSTERIC REPLACEMENTS", PRACTICE OF MEDICINAL CHEMISTRY, XX, XX, 1 January 1996 (1996-01-01), pages 203-237, XP002190259,
- POLYGALOVA, N.N. ET AL.: 'Synthesis of 3,3- dialkyl-1-(3-pyridyl)-3,4-dihydroisoquinoli nes' CHEMISTRY OF HETEROCYCLIC COMPOUNDS vol. 43, no. 8, August 2007, pages 1024 - 1028, XP019550133

## Description

### TECHNICAL FIELD

The present invention relates to a new nitrogen-containing compound and a salt thereof, and a fungicide for agricultural and horticultural use containing at least one of the said compounds.

Priority is claimed on Japanese Patent Application No. 2007-334454, filed December 26, 2007 and Japanese Patent Application No. 2008-032945, filed February 14, 2008.

### BACKGROUND ART

In the cultivation of agricultural and horticultural crops, although a large number of disease control drugs are used against crop disease, since the control effects thereof may be inadequate, the use thereof may be restricted due to the appearance of drug-resistance pathogenic organisms, the plants may be damaged or contaminated by the drug, the drug may demonstrate toxicity to humans, livestock or marine life, or the drug may influence the environment, a considerable number of these control drugs are not necessarily considered to be satisfactory. Thus, there is a need to develop a plant disease control agent that can be used safely and has few of these shortcomings.

In relation to the present invention, a quinoline derivative having a chemical structure similar to the compound of the present invention and a fungicide for agricultural and horticultural use containing the quinoline derivative as an active ingredient are disclosed in Patent Document 1 and Patent Document 2. However, the compound of the present invention is not disclosed.
[Patent Document 1] WO2005/070917
[Patent Document 2] WO2007/011022

FR6806, FR1529059, FR1527576, FR1536543, GB 1174385, GB 1070308 and WO1997/4327 disclose 3,4-dihydro-isoquinoline derivatives which are excluded from the claimed subject-matter by provisos.

### Problems to be Solved by the Invention

An objective of the present invention is to provide a new nitrogen-containing heterocyclic compound and a salt thereof which can be produced industrially advantageously and can be used as an active ingredient of a fungicide for agricultural and horticultural use which shows a definite effect and is safe to use; and a fungicide for agricultural and horticultural use containing at least one of the nitrogen-containing heterocyclic compound and the salt thereof.

### Means for Solving the Problems

In order to achieve the above objective, the first aspect of the present invention provides a nitrogen-containing heterocyclic compound represented by the following formula (I) and a salt thereof.
[Chemical formula 1]

A nitrogen-containing heterocyclic compound represented by the formula (I) 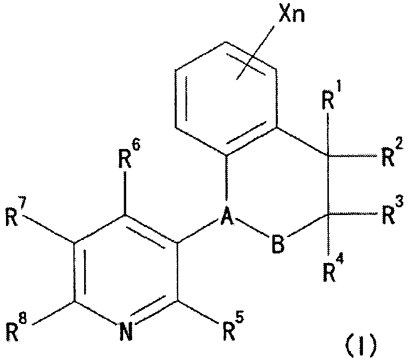 (wherein, A-B represents formulae (II) or (III) 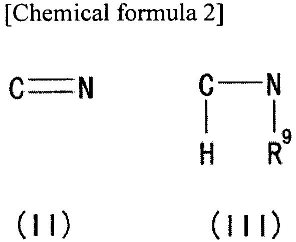
R¹, R², R³ and R⁴ independently represent hydrogen atom, a C1-20 alkyl group, provided that whenA-B represents the formula (II) or (III), R¹, R², R³ and R⁴ do not all represent hydrogen atom;
R⁵ and R⁶ represent a hydrogen atom,
R⁷ and R⁸ independently represent hydrogen atom, a halogen atom, a C1-20 alkyl group, a C1-20 haloalkyl group, a C2-20 alkenyl group, a C2-20 haloalkenyl group, a C2-20 alkynyl group, a C2-20 haloalkynyl group, a C1-20 alkoxy group, a C1-20 haloalkoxy group, a C3-20 cycloalkyl group, a C3-20 cycloalkoxy group, a C2-20 alkenyloxy group, a C2-20 alkynyloxy group, a C1-20 alkyl thio group, cyano group, an unsubstituted or substituted amino group, nitro group, an unsubstituted or substituted phenyl group, an unsubstituted or substituted aralkyl group, an unsubstituted or substituted aryloxy group, an unsubstituted or substituted heterocyclic group, or an unsubstituted or substituted heteroaryloxy group, provided that when A-B represents the formula (II) or (III), R⁷ and R⁸ do not both represent hydrogen atom, and when A-B represents the formula (III) and R⁷ represents hydrogen atom, R⁸ does not represent methyl group;
X represents a halogen atom;
n represents an integer of 0 to 4;
in the formula (III), R⁹ represents hydrogen atom and a salt thereof.

The second aspect of the present invention provides a fungicide for agricultural and horticultural use containing at least one of the compound and the salt thereof according to the present invention.

### Effects of the Invention

The nitrogen-containing heterocyclic compound and the salt thereof of the present invention are new compounds, they can be produced industrially advantageously, and they can be used as an active ingredient of a fungicide for agricultural and horticultural use which is definitely effective and safe to use.

Further, the fungicide for agricultural and horticultural use of the present invention has excellent control effect, does not cause drug disaster or contamination to plants, and demonstrates less toxicity for humans, livestock or marine life and has less environmental impact.

### BEST MODE FOR CARRYING OUT THE INVENTION

The present invention will now be divided into 1) a nitrogen-containing heterocyclic compound represented by the formula (I) and the salt thereof, and 2) a fungicide for agricultural and horticultural use, and be explained below in detail.

### 1) Nitrogen-containing heterocyclic compound represented by the formula (I) and the salt thereof

The first aspect of the present invention is a nitrogen-containing heterocyclic compound represented by the formula (I) and a salt thereof.

In the formula (I), R¹, R², R³ and R⁴ independently represent hydrogen atom, a C1-20 alkyl group, provided that when A-B represents the formula (II) or (III), R¹, R², R³ and R⁴ do not all represent hydrogen atom.

Examples of halogen atom include fluorine atom, chlorine atom, bromine atom and the like.

Examples of C1-20 alkyl group of R¹ to R⁴ include methyl group, ethyl group, n-propyl group, i-propyl group, n-butyl group, i-butyl group, s-butyl group, t-butyl group, n-pentyl group, n-hexyl group, n-heptyl group, n-octyl group, n-decyl group and the like.

Examples of C1-20 haloalkyl group include fluoromethyl group, chloromethyl group, bromomethyl group, difluoromethyl group, dichloromethyl group, dibromomethyl group, trifluoromethyl group, trichloromethyl group, tribromomethyl group, 2, 2, 2-tolufluoroethyl group, 2, 2, 2-trichloroethyl group, pentafluoroethyl group and the like.

Examples of C2 to 20 alkenyl group include vinyl group, 1-propenyl group, 2-propenyl group, 1-butenyl group, 2-butenyl group, 3-butenyl group, 1-methyl-2-propenyl group, 2-methyl-2-propenyl group, 1-pentenyl group, 2-pentenyl group, 3-pentenyl group, 4-pentenyl group, 1-methyl-2-butenyl group, 2-methyl-2-butenyl group, 1-hexenyl group, 2-hexenyl group, 3-hexenyl group, 4-hexenyl group, 5-hexenyl group and the like.

Examples of C2-20 haloalkenyl group include 3-chloro-2-propenyl group, 4-chloro-2-butenyl group, 4, 4-dichloro-3-butenyl group, 4, 4-difluoro-3-butenyl group, 3, 3-dichloro-2-propenyl group and the like.

Examples of C2-20 alkynyl group include ethynyl group, 1-propynyl group, propargyl group, 1-butynyl group, 2-butynyl group, 3-butynyl group, 1-methyl-2-propynyl group, 2-methyl-3-butynyl group, 1-pentynyl group, 2-pentynyl group, 3-pentynyl group, 4-pentynyl group, 1-methyl-2-butynyl group, 2-methyl-3-pentynyl group, 1-hexynyl group, 1, 1-dimethyl-2-butynyl group and the like.

Examples of C2-20 haloalkynyl group include 3-chloro-1-propynyl group, 3-chloro-1-butynyl group, 3-bromo-1-butynyl group, 3-bromo-2-propynyl group, 3-iodo-2-propynyl group and the like.

Examples of C 1-20 alkoxy group include methoxy group, ethoxy group, n-propoxy group, i-propoxy group, n-butoxy group, i-butoxy group, s-butoxy group, t-butoxy group, n-pentyloxy group, n-hexyloxy group and the like.

Examples of C1-20 haloalkoxy group include chloromethoxy group, dichloromethoxy group, trichloromethoxy group, trifluoromethoxy group, 1-fluoroethoxy group, 1, 1-difluoroethoxy group, 2, 2, 2-trifluoroethoxy group, pentafluoroethoxy group and the like.

Examples of C3-20 cycloalkyl group include cyclopropyl group, cyclo-butyl group, cyclopentyl group, cyclohexyl group, cycloheptyl group, cyclooctyl group and the like.

Examples of C4-20 cycloalkenyl group include 1-cyclobutenyl group, 1-cyclopentenyl group, 3-cyclopentenyl group, 1-cyclohexenyl group, 3-cyclohexenyl group, 3-cycloheptenyl group, 4-cyclooctenyl group.

Examples of C8-20 cycloalkynyl group include 5-cyclooctynyl group, 4-cyclodecynyl group and the like.

Examples of C1-20 alkyl carbonyl group include acetyl group, propionyl group, n-propyl carbonyl group, i-propyl carbonyl group, n-butyl carbonyl group, i-butyl carbonyl group, pivaloyl group and the like.

Examples of C1-20 alkoxycarbonyl group include methoxycarbonyl group, ethoxycarbonyl group, n-propoxycarbonyl group, i-propoxycarbonyl group, n-butoxycarbonyl group, i-butoxycarbonyl group, t-butoxycarbonyl group and the like.

Examples of C6-10 aryl group of unsubstituted or substituted C6-10 aryl group include phenyl group, 1-naphthyl group, 2-naphthyl group and the like.

Examples of aralkyl group of unsubstituted or substituted aralkyl group include benzyl group, phenethyl group, 3-phenyl propyl group, 1-naphthyl methyl group, 2-naphthyl methyl group and the like.

Examples of heterocyclic group of unsubstituted or substituted heterocyclic group include a 3 to 7-membered aromatic heterocyclic ring having as atoms constituting the heterocyclic ring 1 to 4 hetero atoms other than carbon atom selected from the group consisting of nitrogen atom, oxygen atom and sulfur atom, a saturated heterocyclic ring, an unsaturated heterocyclic ring and a condensed heterocyclic ring in which said heterocyclic ring and benzene ring are condensed. Specific examples of heterocyclic group include aziridine-1-yl group, aziridine-2-yl group, epoxy group;
tetrahydrofuran-2-yl group, tetrahydrofuran-3-yl group, pyrrolidine-1-yl group, pyrrolidine-2-yl group, pyrrolidine-3-yl group;
pyrrole-1-yl group, pyrrole-2-yl group, pyrrole-3-yl group, furan-2-yl group, furan-3-yl group, thiophene-2-yl group, thiophene-3-yl group, imidazole-1-yl group, imidazole-2-yl group, imidazole-4-yl group, imidazole-5-yl group, pyrazole-1-yl group, pyrazole-3-yl group, pyrazole-4-yl group, pyrazole-5-yl group, oxazole-2-yl group, oxazole-4-yl group, oxazole-5-yl group, thiazole-2-yl group, thiazole-4-yl group, thiazole-5-yl group, isoxazole-3-yl group, isoxazole-4-yl group, isoxazole-5-yl group, isothiazole-3-yl group, isothiazole-4-yl group, isothiazole-5-yl group, 1, 2, 3-triazole-1-yl group, 1, 2, 3-triazole-4-yl group, 1, 2, 3-triazole-5-yl group, 1, 2, 4-triazole-1-yl group, 1, 2, 4-triazole-3-yl group, 1, 2, 4-triazole-5-yl group, 1, 3, 4-oxadiazole-2-yl group, 1, 2, 4-oxadiazole-3-yl group, 1, 3, 4-thiadiazole-2-yl group, 1, 2, 4-thiadiazole-3-yl group, tetrazole-1-yl group, tetrazole-2-yl group;
pyridine-2-yl group, pyridine-3-yl group, pyridine-4-yl group, pyrazine-2-yl group, pyrimidine-2-yl group, pyrimidine-4-yl group, pyrimidine-5-yl group, pyridazine-3-yl group, pyridazine-4-yl group, triazinyl group;
indole-1-yl group, indole-2-yl group, indole-3-yl group, indole-4-yl group, indole-5-yl group, indole-6-yl group, indole-7-yl group, benzofuran-2-yl group, benzofuran-3-yl group, benzofuran-4-yl group, benzofuran-5-yl group, benzofuran-6-yl group, benzofuran-7-yl group, benzothiophene-2-yl group, benzothiophene-3-yl group, benzothiophene-4-yl group, benzothiophene-5-yl group, benzothiophene-6-yl group, benzothiophene-7-yl group, isoindole-1-yl group, isoindole-2-yl group, isoindole-4-yl group, isoindole-5-yl group, isoindole-6-yl group, isoindole-7-yl group, isobenzofuran-1-yl group, isobenzofuran-4-yl group, isobenzofuran-5-yl group, isobenzofuran-6-yl group, isobenzofuran-7-yl group, benzimidazole-1-yl group, benzimidazole-2-yl group, benzimidazole-4-yl group, benzimidazole-5-yl group, benzoxazole-2-yl group, benzoxazole-4-yl group, benzoxazole-5-yl group, benzothiazole-2-yl group, benzothiazole-4-yl group, benzothiazole-5-yl group;
chromene-2-yl group, chromene-3-yl group, chromene-4-yl group, chromene-5-yl group, chromene-6-yl group, chromene-7-yl group, chromene-8-yl group, quinoline-2-yl group, quinoline-3-yl group, quinoline-4-yl group, quinoline-5-yl group, quinoline-6-yl group, quinoline-7-yl group, quinoline-8-yl group, isoquinoline-1-yl group, isoquinoline-3-yl group, isoquinoline-4-yl group, isoquinoline-5-yl group, isoquinoline-6-yl group, isoquinoline-7-yl group, isoquinoline-8-yl group;
piperidine-1-yl group, piperidine-2-yl group, piperidine-3-yl group, piperidine-4-yl group, piperazine-1-yl group, piperazine-2-yl group, piperazine-3-yl group, morpholine-2-yl group, morpholine-3-yl group, morpholine-4-yl group;
1, 3-benzodioxole-4-yl group, 1, 3-benzodioxole-5-yl group, 1, 4-benzodioxane-5-yl group, 1, 4-benzodioxane-6-yl group, 3, 4-dihydro-2H-1, 5-benzodioxepine-6-yl group, 3, 4-dihydro-2H-1, 5-benzodioxepine-7-yl group, 2, 3-dihydrobenzofuran-4-yl group, 2, 3-dihydrobenzofuran-5-yl group, 2, 3-dihydrobenzofuran-6-yl group, 2, 3-dihydrobenzofuran-7-yl group;

Examples of heteroaralkyl group of unsubstituted or substituted heteroaralkyl group include 2-pyridyl methyl group, 3-pyridyl methyl group, 4-pyridyl methyl group, 2-(2-pyridyl)ethyl group, 2-(3-pyridyl)ethyl group, 2-(4-pyridyl)ethyl group, 3-(2-pyridyl)propyl group, 3-(3-pyridyl)propyl group, 3-(4-pyridyl)propyl group, 2-pyradyl emthyl group, 3-pyradyl methyl group, 2-(2-pyradyl)ethyl group, 2-(3-pyradyl)ethyl group, 3-(2-pyradyl)propyl group, 3-(3-pyradyl)propyl group, 2-pyrimidyl methyl group, 4-pyrimidyl methyl group, 2-(2-pyrimidyl)ethyl group, 2-(4-pyrimidyl)ethyl group, 3-(2-pyrimidyl)propyl group, 3-(4-pyrimidyl)propyl group, 2-furyl methyl group, 3-furyl methyl group, 2-(2-furyl)ethyl group, 2-(3-furyl)ethyl group, 3-(2-furyl)propyl group, 3-(3-furyl)propyl group and the like.

Examples of substituent of said C6-10 aryl group, aralkyl group, heterocyclic group and heteroaralkyl group include a halogen atom such as fluorine atom, chlorine atom, bromine atom; a C1-6 alkyl group such as methyl group, ethyl group, n-propyl group, i-propyl group, n-butyl group, s-butyl group, i-butyl group, t-butyl group; a C1-6 alkoxy group such as methoxy group, ethoxy group, n-propoxy group, i-propoxy group, n-butoxy group, s-butoxy group, i-butoxy group, t-butoxy group; a C1-6 haloalkoxy group such as chloromethoxy group, trifluoromethoxy group; a C1-6 alkyl thio group such as methyl thio group, ethyl thio group, n-propyl thio group, i-propyl thio group, n-butyl thio group, i-butyl thio group, s-butyl thio group, t-butyl thio group; a C1-6 alkyl sulfonyl group such as methyl sulfonyl group, ethyl sulfonyl group, n-propyl sulfonyl group, n-butyl sulfonyl group; a C1-6 haloalkyl group such as chloromethyl group, fluoromethyl group, trifluoromethyl group; a C1-6 alkyl sulfonyloxy group such as methyl sulfonyloxy group, ethyl sulfonyloxy group, n-propyl sulfonyloxy group, t-butyl sulfonyloxy group; and the like.

When A-B in the formula (I) represents the formula (II) or (III), R¹ to R⁴ preferably independently represent hydrogen atom or a C1-20 alkyl group.
R⁵ and R⁶ represent a hydrogen atom,
R⁷ and R⁸ independently represent hydrogen atom, a halogen atom, a C1-20 alkyl group, a C1-20 haloalkyl group, a C2-20 alkenyl group, a C2-20 haloalkenyl group, a C2-20 alkynyl group, a C2-20 haloalkynyl group, a C1-20 alkoxy group, a C1-20 haloalkoxy group, a C3-20 cycloalkyl group, a C3-20 cycloalkoxy group, a C2-20 alkenyloxy group, a C2-20 alkynyloxy group, a C1-20 alkyl thio group, cyano group, an unsubstituted or substituted amino group, nitro group, an unsubstituted or substituted phenyl group, an unsubstituted or substituted aralkyl group, an unsubstituted or substituted aryloxy group, an unsubstituted or substituted heterocyclic group, or an unsubstituted or substituted heteroaryloxy group.

Specific examples of a halogen atom, a C1-20 alkyl group, a C1-20 haloalkyl group, a C2-20 alkenyl group, a C2-20 haloalkenyl group, a C2-20 alkynyl group, a C2-20 haloalkynyl group, a C1-20 alkoxy group, a C1-20 haloalkoxy group, an unsubstituted or substituted aralkyl group and an unsubstituted or substituted heterocyclic group of R⁵ R⁷ to R⁸ are the same as the specific examples-described above.

Examples of C3-20 cycloalkyl group of R⁷ to R⁸ include cyclopropyl group, cyclopentyl group, cyclohexyl group and the like.

Examples of C3-20 cycloalcoxy group of R⁷ to R⁸ include cyclopropyloxy group, cyclopentyloxy group, cyclohexyloxy group and the like.

Examples of C2-20 alkenyloxy group of R ⁷ to R⁸ include vinyloxy group, allyloxy group, butenyloxy group and the like.

Examples of C2-20 alkynyloxy group of R⁷ to R⁸ include ethynyloxy group, propynyloxy group, butynyloxy group and the like.

Examples of C1-20 alkylthio group of R⁷ to R⁸ include methyl thio group, ethyl thio group, n-propyl thio group, i-propyl thio group, n-butyl thio group, t-butyl thio group and the like.

Examples of unsubstituted or substituted amino group of R⁷ to R⁸ include amino group; a mono C1-C6 alkyl amino group such as methyl amino group, ethyl amino group; a di C1-C6 alkyl amino group such as dimethyl amino group, diethyl amino group; an acyl amino group such as acetyl amino group, benzoyl amino group; an unsubstituted or substituted phenyl amino group such as phenyl amino group, 4-methyl phenyl amino group; and the like.

Examples of aryloxy group of unsubstituted or substituted aryloxy group of R⁷ to R⁸ include phenoxy group, 1-naphthyloxy group, 2-naphthyloxy group and the like.

Examples of heteroaryloxy group of unsubstituted or substituted heteroaryloxy group of R⁷ to R⁸ include imidazole-2-yloxy group, imidazole-4-yloxy group, oxazole-2-yloxy group, thiazole-2-yloxy group, isoxazole-3-yloxy group, isothiazole-3-yloxy group, 2-furyloxy group, 2-thienyloxy group, 2-pyridyloxy group, 3-pyridyloxy group, 4-pyridyloxy group, pyrimidine-2-yloxy group, pyrazine-3-yloxy group and the like.

Examples of substituent of said unsubstituted or substituted phenyl group, unsubstituted or substituted aryloxy group, unsubstituted or substituted heterocyclic group, unsubstituted or substituted heterocyclic aryloxy group are the same as the examples of said substituent of unsubstituted or substituted C6-10 aryl group described above.

Among these, R⁷ to R⁸ are preferably independently hydrogen atom, a halogen atom, a C1-20 alkyl group, a C1-20 haloalkyl group or a C1-20 alkoxy group.

In addition, R⁵ and/or R⁶ are hydrogen atom.

However, when A-B represents the formula (II) or (III), R⁷ and R⁸ do not both represent hydrogen atom, and when A-B represents the formula (III) and R⁷ represents hydrogen atom, R⁸ does not represent methyl group.

X represents a halogen atom such as fluorine atom, chlorine atom, bromine atom.

n represents an integer of 0 to 4.

In the formula (III), R⁹ represents hydrogen atom.

The nitrogen-containing heterocyclic compound of the present invention may be produced by the production methods described below. (Refer to J. Heterocyclic Chem., 24, 351(1987), publication of EP1375496)

### (Production Method 1)

Among the compounds represented by the formula (I), a compound represented by the formula (Ia) (hereinafter, to be referred to as "compound (Ia)") can be produced by reacting a compound represented by the formula (1) below with an alcohol compound represented by the formula (2) below in the presence of an acid in a solvent or under solvent-free conditions

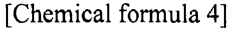

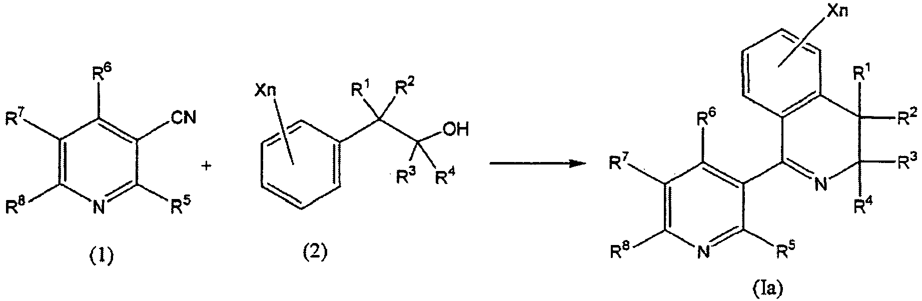

(In the formula, X, n, R¹ to R⁸ are as defined above.)

The used amount of the alcohol compound represented by the formula (2) is normally 1 to 6 moles, preferably 1.1 to 3 moles with respect to 1 mole of the compound represented by the formula (1).

Examples of the acid to be used in the production method include an inorganic acid such as hydrochloric acid, sulfuric acid, nitric acid, phosphoric acid, perchloric acid; a carboxylic acid such as formic acid, acetic acid, trifluoroacetic acid;
a sulfonic acid such as benzene sulfonic acid, p-toluene sulfonic acid, trifluoromethane sulfonic acid; Lewis acid such as tin(IV) chloride, trifluoroboron; and the like.

The amount of the acid to be used is normally 1 to 50 moles, preferably 1.1 to 30 moles with respect to 1 mole of the compound represented by the formula (1).

The solvent to be used is not particularly limited as long as it is inactive against the reaction. For example, aromatic hydrocarbons such as benzene, toluene, xylene; an aliphatic hydrocarbon such as n-hexane, n-heptane; alicyclic hydrocarbons such as cyclohexane; aromatic halogenated hydrocarbons such as chlorobenzene, dichlorobenzene; aliphatic halogenated hydrocarbons such as dichloromethane, chloroform, carbon tetrachloride, 1, 2-dichloroethane, 1, 1, 1-trichloroethane, trichloroethylene, tetrachloroethylene; ethers such as diethyl ether, 1,2-dimethoxyethane, tetrahydrofuran, 1, 4-dioxane; and the like.

These solvents may be used alone or in combinations of two or more.

The reaction temperature is normally -60 °C to +100 °C, preferably 0 °C to 80 °C.

The reaction time is normally from a few minutes to 120 hours, preferably 30 minutes to 70 hours.

Each of the compound represented by the formula (1) which is a starting material and the alcohol compound represented by the formula (2) can be produced by a well-known production method (refer to J. Med. Chem., (1979) Vol. 22, 816, Tetrahedron, Vol. 55, 4595 and the like).

### (Production Method 2)

Furthermore, from a compound (Ia) wherein any of R⁵ to R⁸ represents a halogen atom, a compound of the present invention wherein said halogen atom is converted to other substituent can be produced. The conversion of halogen atom to other substituent can be achieved by a well-known method.

For example, from a compound represented by a formula (Ia') wherein R⁸ represents a halogen atom (in the formula, R^{8'} represents a halogen atom such as chlorine atom, bromine atom or the like. Hereinafter, to be referred to as "compound (Ia')"), a compound represented by a formula (Ia") (in the formula, R^{8"} represents the same as that of R⁸ except halogen atom. Hereinafter, to be referred to as "compound (Ia")") can be produced.

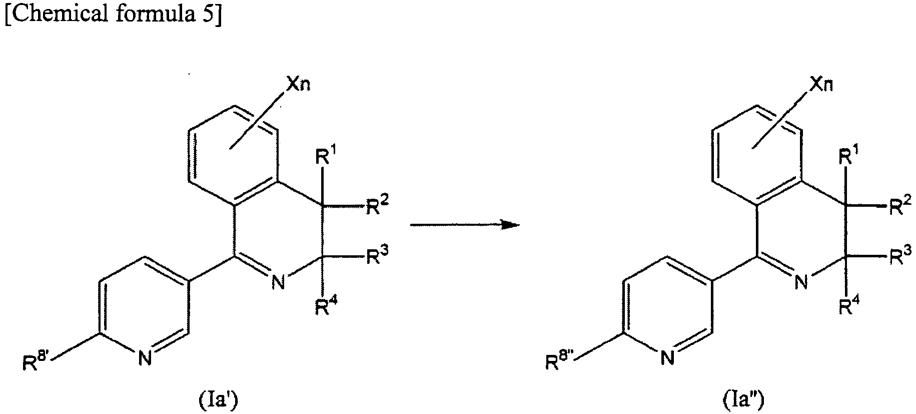

(In the formula, X, n, R¹ to R⁴ are defined as described above.)

More specifically, the compound (Ia") can be produced by the method described below.
i) the compound (Ia") wherein R⁸is R^{8"} (R^{8"} represents the same as described above) can be obtained by reacting the compound (Ia') with a grignard reagent represented by R^{8"} MgX' (X' represents halogen atom) or an organic zinc compound represented by R^{8"}ZnCl.
ii) the compound (Ia") wherein R⁸is R^{8"} can be obtained by reacting the compound (Ia') with a metallic alkoxide represented by MOR^{8"} (M represents alkali metal).
iii) the compound (Ia") wherein R^{8"} represents cyano group can be obtained by reacting the compound (Ia') with zinc cyanide in the presence of tetra(triphenyl phosphino)palladium [Pd(PPh₃)₄].
iv) the compound (Ia") wherein R^{8"} represents phenyl group can be obtained by reacting the compound (Ia') with phenyl boronic acid in the presence of Pd(PPh₃)₄ and a base.

### (Production Method 3)

Among the compounds represented by the formula (I), a compound represented by a formula (Ib) (Hereinafter, to be referred to as "compound (Ib)") can be obtained by reduction of the compound (Ia) (refer to the reaction formula below). 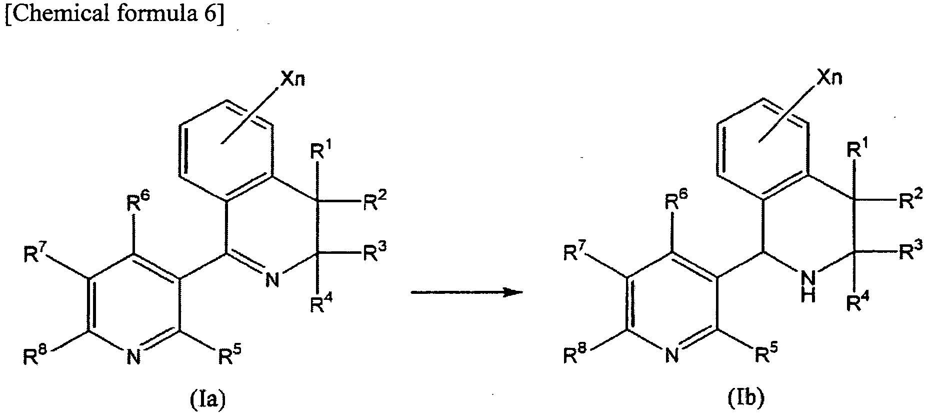 (in the formula, X, n, R¹ to R⁸ are defined as described above.)

The reduction method is not particularly limited and a well-known method can be used as the reduction method. For example, (i) acatalytic hydrogenation method using hydrogen and a catalytic reduction catalyst such as palladium-carbon, platinum oxide, raney nickel or the like, (ii) a reduction method using a metallic hydride such as lithium aluminium hydride, sodium boron hydride, sodium cyanoborohydride or the like.

The used amount of the catalyst in the method (i) is normally 0.001 to 10 moles with respect to 1 mole of the compound (Ia). Further, hydrogen pressure is normally 1 to 10 atmospheres.

The used amount of the metallic hydride in the method (ii) is normally 1 to 20 moles, preferably 1 to 10 moles with respect to 1 more of the compound (Ia).

Either reaction is preferably performed in a solvent. The solvent to be used is not particularly limited as long as the solvent is inactive against the reaction. For example, aromatic hydrocarbons such as benzene, toluene, xylene; an aliphatic hydrocarbon such as n-hexane, n-heptane; alicyclic hydrocarbons such as cyclohexane; aromatic halogenated hydrocarbons such as chlorobenzene, dichlorobenzene; aliphatic halogenated hydrocarbons such as dichloromethane, chloroform, carbon tetrachloride, 1, 2-dichloroethane, 1, 1, 1-trichloroethane, trichloroethylene, tetrachloroethylene; ethers such as diethyl ether, 1, 2-dimethoxyethane, tetrahydrofuran, 1, 4-dioxane; alcohols such as methanol, ethanol, ethylene glycol; carboxylic acids such as acetic acid, propionic acid; inorganic acid such as hydrochloric acid, sulfuric acid; water; and the like.

These solvents may be used alone or in combinations of two or more.

The reaction temperature is normally 0°C to 200 °C, preferably 20 °C to 180 °C. The reaction time is normally from a few minutes to 120 hours, preferably 2 to 72 hours.

The salt of the compound represented by the formula (I) is not particularly limited as long as it is permitted from an agricultural and horticultural perspective. Examples which may be cited include an inorganic acid salt such hydrochloride salt, nitrate salt, sulfate salt, phosphoric acid of the compound represented by the formula (I); a salt of an organic acid such as acetic acid, propionic acid and lactic acid of the compound represented by the formula (I); and the like.

The salt of the compound represented by the formula (I), for example, can be produced by the action of inorganic acid or organic acid on the compound represented by the formula (I).

In either of these reactions, if purification of the product is required after the completion of the reaction, known, commonly used purification means, such as distillation, recrystallization or column chromatography, can be employed following carrying out an ordinary post-treatment operation.

The structure of a target compound can be identified and confirmed by known analysis means such as elementary analysis, NMR spectroscopy, IR spectroscopy or mass spectrometry.

Examples of the nitrogen-containing heterocyclic compound of the present invention that is obtained in a manner described above are shown in Tables 1 to 2 below. in the following tables, Me represents methyl group, Et represents ethyl group, c-Pr represent a cyclopropyl group, Py represents pyridyl group, Fr represents furyl group, Im represents imidazolyl group, Th represent thienyl group, Ph represents phenyl group, Ac represents acetyl group.

**Table 1**

| | | | | | | | | | |
|---|---|---|---|---|---|---|---|---|---|
| 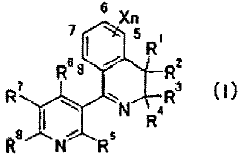 | | | | | | | | | |

| No. | R¹ | R² | R³ | R⁴ | R⁵ | R⁶ | R⁷ | R⁸ | Xn |
|---|---|---|---|---|---|---|---|---|---|
| Ia-1 | H | H | Me | Me | H | H | Me | H | - |
| Ia-2 | H | H | Me | Me | H | H | Et | H | - |
| Ia-3 | H | H | Me | Me | H | H | n-Pr | H | - |
| Ia-4 | H | H | Me | Me | H | H | CH=CH₂ | H | - |
| Ia-5 | H | H | Me | Me | H | H | Br | H | - |
| Ia-6 | H | H | Me | Me | H | H | CF₃ | Me | - |
| Ia-7 | H | H | Me | Me | H | H | CF₃ | Me | 5-F |
| Ia-8 | H | H | Me | Me | H | H | H | Et | - |
| Ia-9 | H | H | Me | Me | H | H | H | n-Pr | - |
| Ia-10 | H | H | Me | Me | H | H | H | i-Pr | - |
| Ia-11 | H | H | Me | Me | H | H | H | Cl | - |
| Ia-12 | H | H | Me | Me | H | H | H | CH=CH₂ | - |
| Ia-13 | H | H | Me | Me | H | H | H | Ph | - |
| Ia-14 | H | H | Me | Me | H | H | H | CN | - |
| Ia-15 | H | H | Me | Me | H | H | Me | Me | - |
| Ia-16 | H | H | Me | Me | H | H | Me | Me | 5-F |
| Ia-17 | H | H | Me | Me | H | H | Me | Et | - |
| Ia-18 | H | H | Me | Me | H | H | Et | Me | - |
| Ia-19 | H | H | Me | Me | H | H | Cl | Cl | - |
| Ia-20 | H | H | Me | Me | H | H | Cl | Et | - |
| Ia-21 | H | H | Me | Me | H | H | Cl | OMe | - |
| Ia-23 | Me | Me | Me | Me | H | H | Me | Me | 5-F |
| Ia-24 | H | H | Me | Me | H | H | H | CH₂C≡CH | - |
| Ia-25 | H | H | Me | Me | H | H | H | SMe | - |
| Ia-26 | H | H | Me | Me | It | H | CF₃ | Me | 6-F |
| Ia-27 | H | H | Me | Me | H | H | CF₃ | Me | 7-F |
| Ia-28 | H | H | Me | Me | H | H | CF₃ | Me | 8-F |
| Ia-29 | H | H | Me | Me | H | H | H | CF₃ | - |
| Ia-30 | H | H | Me | Me | H | H | H | OPh | - |
| Ia-31 | H | H | Me | Me | H | H | OCF₃ | H | - |
| Ia-32 | H | H | Me | Me | H | H | NH₂ | H | - |
| Ia-33 | H | H | Me | Me | H | H | NO₂ | H | - |
| Ia-34 | H | H | Me | Me | H | H | Ph | H | - |
| Ia-58 | H | H | Me | Me | H | H | Me | CF₃ | 5-F |
| Ia-59 | H | H | Me | Me | H | H | Me | C₂F₅ | 5-F |
| Ia-60 | H | H | Me | Me | H | H | Me | NHAc | 5-F |

**Table 2**

| | | | | | | | | | | |
|---|---|---|---|---|---|---|---|---|---|---|
| 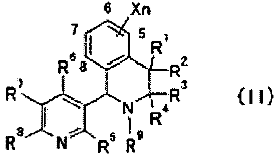 | | | | | | | | | | |

| No. | R¹ | R² | R³ | R⁴ | R⁵ | R⁶ | R⁷ | R⁸ | R⁹ | Xn |
|---|---|---|---|---|---|---|---|---|---|---|
| Id-1 | H | H | Me | Me | H | H | Me | Me | H | 5-F |
| Id-2 | Me | Me | Me | Me | H | H | Me | Me | H | 5-F |
| Id-3 | H | H | Me | Me | H | H | Me | H | H | - |
| Id-4 | H | H | Me | Me | H | H | Et | H | H | - |
| Id-5 | H | H | Me | Me | H | H | n-Pr | H | H | - |
| Id-6 | H | H | Me | Me | H | H | CH=CH₂ | H | H | - |
| Id-7 | H | H | Me | Me | H | H | Br | H | H | - |
| Id-8 | H | H | Me | Me | H | H | H | Et | H | - |
| Id-9 | H | H | Me | Me | H | H | H | n-Pr | H | - |
| Id-10 | H | H | Me | Me | H | H | H | i-Pr | H | - |
| Id-11 | H | H | Me | Me | H | H | H | Cl | H | - |
| Id-12 | H | H | Me | Me | H | H | H | CH=CH₂ | H | - |
| Id-13 | H | H | Me | Me | H | H | H | Ph | H | - |
| Id-14 | H | H | Me | Me | H | H | H | CN | H | - |
| Id-15 | H | H | Me | Me | H | H | Me | Et | H | - |
| Id-16 | H | H | Me | Me | H | H | Cl | Cl | H | - |
| Id-17 | H | H | Me | Me | H | H | Cl | Et | H | - |
| Id-18 | H | H | Me | Me | H | H | Cl | OMe | H | - |
| Id-20 | H | H | Me | Me | H | H | H | CF₃ | Me | - |
| Id-21 | H | H | Me | Me | H | H | H | CH₂C≡CH | Et | - |
| Id-22 | H | H | Me | Me | H | H | H | SMe | i-Pr | - |
| Id-23 | H | H | Me | Me | H | H | H | OPh | Me | - |
| Id-24 | H | H | Me | Me | H | H | OCF₃ | Et | Et | - |
| Id-25 | H | H | Me | Me | H | H | NH₂ | n-Pr | Et | - |
| Id-26 | H | H | Me | Me | H | H | Ph | Cl | Me | - |
| Id-27 | H | H | Me | Me | H | H | Me | H | H | 5-F |
| Id-28 | H | H | Me | Me | H | H | Me | H | H | 5-F |
| Id-29 | H | H | Me | Me | H | H | Me | H | H | 6-F |
| Id-30 | H | H | Me | Me | H | H | Me | H | H | 7-F |
| Id-31 | H | H | Me | Me | H | H | Me | H | H | 8-F |
| Id-54 | H | H | Me | Me | H | H | Me | CF₃ | H | 5-F |
| Id-55 | H | H | Me | Me | H | H | Me | C₂F₆ | H | 5-F |
| Id-56 | H | H | Me | Me | H | H | Me | NHAc | H | 5-F |

### 2) Fungicide for Agricultural and Horticultural Use

The second aspect of the present invention is a fungicide for agricultural and horticultural use containing at least one of the nitrogen-containing heterocyclic compound represented by the formula (I) and the salt thereof (hereinafter, to be referred to as "fungicide of the present invention").

The content of active ingredient is normally 0.01 to 90% by weight, preferably 0.05 to 85% by weight with respect to the total content of the fungicide.

The fungicide of the present invention has superior bactericidal action against a wide range of types of fungi, such as fungi belonging to Oomycetes, Ascomycetes, Deuteromycetes and Basidiomycetes.

The compound containing the fungicide of the present invention as an active ingredient can be used to control various plant diseases occurring during cultivation of agricultural and horticultural crops such as flowering plants, lawn grasses and pasture grasses by seed treatment, foliar spraying, soil application or water surface application and the like.

Examples of crops in which plant diseases can be controlled along with their plant diseases and causative organisms include:
Sugar Beets:
   Cercospora leaf spot (Cercospora beticola)
Peanuts:
   Brown leaf spot (Mycosphaerella arachidis)
   Black leaf blight (Mycosphaerella berkeleyi)
Cucumbers:
   Powdery mildew (Sphaerotheca fuliginea)
   Gummy stem blight (Mycosphaerella melonis)
   Sclerotinia rot (Sclerotinia sclerotiorum)
   Gray mold (Botrytis cinerea)
   Scab (Cladosporium cucumerinum)
   Corynespora leaf spot (Corynespora cassicola)
   Damping-off (Pythium debaryanam, Rhizoctonia solani Kuhn)
   Bacterial spot (Pseudomonas syringae pv. Lecrymans)
Tomatoes:
   Gray mold (Botrytis cinerea)
   Leaf mold (Cladosporium fulvum)
Eggplants:
   Gray mold (Botrytis cinerea)
   Black rot (Corynespora malongenae)
   Powdery mildew (Erysiphe cichoracearum)
   Leaf mold (Mycovellosiella nattrassii)
Strawberries:
   Gray mold (Botrytis cinerea)
   Powdery mildew (Sphaerotheca humuli)
   Anthracnose (Colletotrichum acutatum, Colletotrichum fragariae)
Onions:
   Neck rot (Botrytis allii)
   Gray mold (Botrytis cinerea)
   Leaf blight (Botrytis squamosa)
Cabbage:
   Clubroot (Plasmodiophora brassicae)
   Bacterial soft rot (Erwinia carotovora)
Kidney beans:
   Stem rot (Sclerotinia sclerotiorum)
   Gray mold (Botrytis cinerea)
Apples:
   Powdery mildew (Podosphaera leucotricha)
   Scab (Venturia inaequalis)
   Blossom blight (Monilinia mali)
   Valsa canker (Valsa mali)
   Alternaria blotch (Alternaria mali)
   Rust (Gymnosporangium yamadae)
   Ring rot (Botryosphaeria berengeriana)
   Anthracnose (Coletotrichum gloeosprioides)
   Blotch (Diplocarpon mali)
Persimmons:
   Powdery mildew (Phyllactinia kakicola)
   Anthracnose (Gloeosporium kaki)
   Angular leaf spot (Cercospora kaki)
Peaches and cherries:
   Brown rot (Monilinia fructicola)
Grapes:
   Gray mold (Botrytis cinerea)
   Powdery mildew (Uncinula necator)
   Ripe rot (Glomerella cingulata)
Pears:
   Scab (Venturia nashicola)
   Rust (Gymnosporangium asiaticum)
   Black spot (Alternaria kikuchiana)
Tea:
   Gray blight (Pestalotia theae)
   Anthracnose (Collectotrichum theae-sinensis)
Citrus:
   Scab (Elsinoe fawcette)
   Blue mold (Penicillium italicum)
   Common green mold (Penicillium digitatum)
   Gray mold (Botrytis cinerea)
   Melanose (Diaporthe citri)
   Canker (Xanthomonas campestris pv. Citri)
Wheat:
   Powdery mildew (Erysiphe graminis f. sp. tritici)
   Fusarium blight (Gibberella zeae)
   Leaf rust (Puccinia recondita)
   Browining root rot (Pythium iwayamai)
   Snow mold (Monographella nivalis)
   Eye spot (Pseudocercosporella herpotrichoides)
   Speckled leaf blotch (Septoria tritici)
   Glume blotch (Leptosphaeria nodorum)
   Typhula snow blight (Typhula incarnata)
   Sclerotinia snow blight (Myriosclerotinia borealis)
   Take-all (Gaeumanomyces graminis)
Barley:
   Stripe (Pyrenophora graminea)
   Leaf blotch (Rhynchosporium secalis)
   Loose smut (Ustilago tritici, U. nuda)
Rice:
   Blast (Pyricularia oryzae)
   Sheath blight (Rhizoctonia solani)
   Bakanae disease (Gibberella fujikuroi)
   Brown spot (Cochliobolus niyabeanus)
Tobacco:
   Sclerotinia stem-rot (Sclerotinia sclerotiorum)
   Powdery mildew (Erysiphe cichoracearum)
Tulips:
   Gray mold (Botrytis cinerea)
Bent grass:
   Sclerotinia snow blight (Sclerotinia borealis)
Orchard grass:
   Powdery mildew (Erysiphe graminis)
Soybeans:
   Purple stain (Cercospora kikuchii)
Potatoes, tomatoes:
   Late blight (Phytophthora infestans)
Cucumbers:
   Downy mildew (Pseudoperonospora cubensis)
Grapes:
   Downy mildew (Plasmopara viticola)

In addition, various pathogens have recently developed resistance to benzimidazole fungicides and dicarboximide fungicides resulting in inadequate efficacy of these drugs, thereby creating the need for effective drugs against resistant organisms as well. The fungicide of the present invention also has superior bactericidal effects against resistant organisms in addition to pathogens that are sensitive to these drugs.

For example, the fungicide of the present invention is effective against gray mold (Botrytis cinerea), sugar beet cercospora leaf spot (Cercospora beticola) and apple scab (Venturia inaequalis), pear scab (Venturia nashicola), which exhibit resistance to benzimidazole fungicides such as thiophanate-methyl, benomyl, carbendazim and thiabendazole, in the same manner as sensitive organisms.

Moreover, the fungicide of the present invention is effective against gray mold (Botrytis cinerea), which exhibits resistance to dicarboximide fungicides (such as vinclozoline, procymidone and iprodione) in the same manner as sensitive organisms.

Examples of diseases for which application of the fungicide of the present invention is more preferable include sugar beet cercospora leaf spot, wheat powdery mildew, rice blast, apple scab, cucumber gray mold and peanut brown leaf spot.

In addition, the fungicide of the present invention causes little chemical damage, exhibits low toxicity to fish and warm-blooded animals, and has a high degree of safety.

The compound of the present invention may be used in pure form without adding other components, or may be used in the form able to be adopted by an ordinary agricultural chemical, such as a wettable powder, granules, powder, emulsion, aqueous solution, suspension or water-dispersible granules using an additive or a support.

Examples of additives and carriers able to be added to the agricultural chemical preparation used for the purpose of solid formulations include vegetable powders such as soybean powder or wheat powder, mineral fine powders such as diatomaceous earth, apatite, gypsum, talc, bentonite, pyrophyllite or clay, and organic and inorganic compounds such as sodium benzoate, urea or sodium sulfate.

In addition, in the case of using for the purpose of liquid formulations, kerosene, xylene and petroleum-based aromatic hydrocarbons, cyclohexane, cyclohexanone, dimethylformamide, dimethylsulfoxide, alcohol, acetone, trichloroethylene, methyl isobutyl ketone, mineral oil, vegetable oil and water, for example, can be used as solvents.

Moreover, a surfactant can be added to these preparations as necessary to obtain a uniform and stable form.

There are no particular limitations on surfactants to be used, and examples include nonionic surfactants such as polyoxyethylene-alkyl phenyl ethers, polyoxyethylene-alkyl ethers, polyoxyethylene-higher fatty acid esters, polyoxyethylene-sorbitan fatty acid esters or polyoxyethylene-tristyryl phenyl ether, and sulfuric acid ester salts of polyoxyethylene-alkyl phenyl ethers, alkyl benzene sulfonates, sulfuric acid ester salts of higher alcohols, alkyl naphthalene sulfonates, polycarboxylates, lignin sulfonates, formaldehyde condensates of alkyl naphthalene sulfonates and isobutylene-maleic anhydrate copolymers.

Wettable powders, emulsions, flowable agents, aqueous solutions and water-dispersible granules are used in the form of solutions, suspensions or emulsions by diluting to a prescribed concentration with water, while powders and granules are used by spraying directly onto plants.

The formulated fungicide of the present invention may be applied directly onto plants, seeds, the surface of water or soil, or applied after diluting with water.

Although the applied amount of the fungicide varies according to weather conditions, preparation form, application time, application method, applied location, target control disease, target crop and the like, it is normally 1 to 1, 000 g and preferably 10 to 100 g as the amount of active ingredient compound per hectare.

In the case of applying by diluting a wettable powder, emulsion, suspension, aqueous solutions and water-dispersible granules with water, the applied concentration is 1 to 1000 ppm and preferably 10 to 250 ppm. In the case of granular formulation, dust formulation or the like, it may be applied directly without diluting.

In addition to the compound of the present invention, the fungicide of the present invention can also be mixed with one or two or more of various fungicides, insecticides, miticides or synergists.

Typical examples of fungicides, insecticides, miticides and plant growth regulators able to be used by mixing with the compound of the present invention are indicated below.

Fungicides:
   captan, folpet, thiuram, ziram, zineb, maneb, mancozeb, propineb, polycarbamate, chlorothalonil, quintozene, captafol, iprodione, procymidone, vinclozolin, fluoroimide, cymoxanil, mepronil, flutolanil, pencycuron, oxycarboxin, fosetyl-aluminum, propamocarb, triadimefon, triadimenol, propiconazole, diclobutorazol, bitertanol, hexaconazol, miclobutanil, flusilazole, metconazole, etaconazole, fluotrimazole, cyproconazole, epoxyconazole, flutriafen, penconazole, diniconazole, cyproconazole, fenarimol, triflumizole, prochloraz, imazalil, pefurazoate, tridemorph, fenpropimorph, triforine, buthiobate, pyrifenox, anilazine, polyoxin, metalaxyl, oxadixyl, furalaxyl, isoprothiolane, probenazole, pyrrolnitrin, blasticidin S, kasugamycin, validamycin, dihydrostreptomycin sulfate, benomyl, carbendazim, thiophanate-methyl, hymexazol, basic copper chloride, basic copper sulfate, fentin acetate, triphenyltin hydroxide, diethofencarb, methasulfocarb, qinomethionate, binapacryl, lecithin, sodium bicarbonate, dithianon, dinocap, fenaminosulf, diclomezine, guazatine, dodine, IBP, edifenphos, mepanipyrim, ferimzone, trichlamide, methasulfocarb, fluazinam, ethoqinolac, dimethomorph, pyroquilon, tecloftalam, fthalide, phenazine oxide, thiabendazole, tricyclazole, vinclozolin, cymoxanil, cyclobutanil, guazatine, propamocarb hydrochloride, oxolinic acid, hydroxyisoxazole, iminoctadine acetate, and the like.
Insecticides/Miticides:
   Organic phosphorous and carbamate-based insecticides:
      fenthion, fenitrothion, diazinon, chlorpyrifos, ESP, vamidothion, phenthoate, dimethoate, formothion, malathion, trichlorfon, thiometon, phosmet, dichlorvos, acephate, EPBP, methyl parathion, oxydemetone methyl, ethion, salithion, cyanophos, isoxathion, pyridafenthion, phosalone, methidathion, sulprofos, chlorfenvinphos, tetrachlorovinphos, dimethylvinphos, propaphos, isofenphos, ethyl thiometon, profenofos, pyraclofos, monocrotophos, azinphos-methyl, aldicarb, methomyl, thiodicarb, carbofuran, carbosulphan, benfuracarb, furathiocarb, propoxur, BPMC, MTMC, MIPC, carbaryl, pirimicarb, ethiophencarb, phenoxycarb, EDDP, and the like.
Pyrethroid-based insecticides:
   permethrin, cypermethrin, deltamethrin, fenvalerate, fenpropathrin, pyrethrin, allethrin, tetramethrin, resmethrin, dimethrin, propathrin, phenothrin, prothrin, fluvalinate, cyfluthrin, cyhalothrin, flucythrinate, etofenprox, cycloprothrin, tralomethrin, silafluofen, flufenprox, acrinathrin, and the like.
Benzoylurea-based and other insecticides: diflubenzuron, chlorfluazuron, hexaflumuron, triflumuron, tetrabenzuron, flufenoxuron, flucycloxuron, buprofezin, pyriproxyfen, methoprene, benzoepin, diafenthiuron, acetamiprid, imidacloprid, nitenpyram, fipronil, cartap, thiocyclam, bensultap, nicotine sulfate, rotenone, metaldehyde, machine oil, BT and microbial agrichemicals such as insect pathogenic viruses.
Nematocides:
   Fenamiphos, fosthiazate, and the like
Miticides:
   Chlorobenzilate, phenisobromolate, dicofol, amitraz, BPPS, benzomate, hexythiazox, fenbutatin oxide, polynactin, chinomethionate, CPCBS, tetradifon, avermectin, milbemectin, clofentezine, cyhexatin, pyridaben, fenpyroximate, tebufenpyrad, pyrimidifen, fenothiocarb, dienochlor, and the like.
Plant growth regulators:
   Gibberellins (such as gibberellin A3, gibberellin A4 or gibberellin A7), IAA, NAA.

### EXAMPLES

Although the following provides a more detailed explanation of the present invention through examples thereof, the present invention is not limited to the following examples. The compound numbers described blow correspond to the numbers described in Tables 1 to 2.

The following are referenced documents in the examples below.
(1) Synthesis of 2, 3-dialkyl-5-cyanopyridine J. Heterocyclic Chem., 24, 351 (1987)
(2) Alkylation of pyridine Patent document of EP1375496

### (Example 1)

### Production of 1-(5-bromopyridine-3-yl)-3, 3-dimethyl-3, 4-dihydroisoquinoline (compound number: Ia-5)

Under ice-cold conditions, 2.80 g (15.0 mmol) of 3-bromo-5-cyanopyridine was added to 15mL of concentrated sulfuric acid, and a further 2.76 g (17.7 mmol) of 2-methyl-3-phenyl-2-propanol was dropped into the mixture slowly followed by stirring for 2 hours at room temperature. The reaction solution was poured into ice water and neutralized with sodium carbonate followed by extraction with ethyl acetate. The organic layer was washed with water and dried by addition of anhydrous magnesium sulfate followed by distilling off the solvent under reduced pressure. The resulting residue was purified by silica gel column chromatography (developing solvent: n-hexane:ethyl acetate = 9:1 (volume ratio)) to obtain 3.2 g (yield: 68%) of the target compound. The obtained compound was amorphous. ¹H-NMR data of the compound are shown below.

¹H-NMR(300MHz, CDCl₃)δppm; 1.28(6H, s), 2.82(2H, s), 7.14(1H, d, J=7.2Hz), 7.2-7.3(2H, m), 7.3-7.5(1H, m), 8.07(1H, m), 8.70(1H, d, J=1.8Hz), 8.73(1H, d, J=2.1Hz)

The compounds produced in the same manner as Example 1 are shown in below.
¹H-NMR data of the obtained compounds are shown respectively.

### (Compound number: Ia-1) 1-(5-methyl pyridine-3-yl)-3, 3-dimethyl-3, 4-dihydroisoquinoline (physical property: amorphous)

¹H-NMR(300MHz, CDCl₃) δ ppm; 1.29(6H, s), 2.39(3H, s), 2.82(2H, s), 7.1-7.3(4H, m), 7.3-7.45(1H, m), 7.72(1H, m), 8.50(1H, d, J=1.8Hz), 8.57(1H, d, J=1.8Hz)

### (Compound number: Ia-11) 1-(6-chloropyridine-3-yl)-3, 3-dimethyl-3, 4-dihydroisoquinoline (physical property: amorphous)

¹H-NMR(300MHz, CDCl₃) δ ppm; 1.28(6H, s), 2.81(2H, s), 7.13(1H, d, J=7.5Hz), 7.2-7.3(2H, m), 7.3-7.5(2H, m), 7.8-7.9(1H, m), 8.58(1H, d, J=2.4Hz)

### (Compound number: Ia-15) 1-(5, 6-dimethyl pyridine-3-yl)-3, 3-dimethyl-3, 4-dihydroisoquinoline (physical property: amorphous)

¹H-NMR(300MHz, CDCl₃) δ ppm; 1.27(6H, s), 2.33(3H, s), 2.55(3H, s), 2.80(2H, s), 7.1-7.3(3H, m), 7.3-7.5(1H, m), 7.65(1H, d, J=1.8Hz), 8.46(1H, d, J=1.8Hz)

### (Compound number: Ia-16) 1-(5, 6-dimethyl pyridine-3-yl)-3, 3-dimethyl-5-fluoro-3, 4-dihydroisoquinoline (physical property: amorphous)

¹H-NMR(300MHz, CDCl₃) δ ppm; 1.30(6H, s), 2.33(3H, s), 2.55(3H, s), 2.82(2H, s), 7.01(1H, dd, J=1.8, 7.2Hz), 7.1-7.3(2H, m), 7.64(1H, s), 8.44(1H, s)

### (Compound number: Ia-17) 1-(6-ethyl-5-methyl pyridine-3-yl)-3, 3-dimethyl-3, 4-dihydroisoquinoline (physical property: amorphous)

¹H-NMR(300MHz, CDCl₃) δ ppm; 1.27(3H, t, J=7.5Hz), 1.29(6H, s), 2.36(3H, s), 2.80(2H, s), 2.86(2H, q, J=7.5Hz), 7.2-7.3(3H, m), 7.38(1H, m), 7.65(1H, s), 8.50(1H, s)

### (Compound number: Ia-18) 1-(5-ethyl-6-methyl pyridine-3-yl)-3, 3-dimethyl-3, 4-dihydroisoquinoline (physical property: amorphous)

¹H-NMR(300MHz, CDCl₃) δ ppm; 1.26(3H, t, J=7.8Hz), 1.29(6H, s), 2.59(3H, s), 2.69(2H, q, J=7.5Hz), 2.82(2H, s), 7.1-7.3(3H, m), 7.39(1H, dt, J=1.5, 7.0Hz), 7.64(1H, d, J=2.3Hz), 8.49(1H, d, J=2.3Hz)

### (Compound number: Ia-19) 1-(5, 6-dichloropyridine-3-yl)-3, 3-dimethyl-3, 4-dihydroisoquinoline (physical property: amorphous)

¹H-NMR(300MHz, CDCl₃) δ ppm; 1.27(6H, s), 2.81(2H, s), 7.1-7.5(4H, m), 8.04(1H, s), 8.47(1H, s)

### (Reference compound Ia-22) 1-(2-chloro-5, 6-dimethyl pyridine-3-yl)-3, 3-dimethyl-3, 4-dihydroisoquinoline (physical property: amorphous)

¹H-NMR(300MHz, CDCl₃) δ ppm; 1.0-1.5(6H, bs), 2.30(3H, s), 2.53(3H, s), 2.84(2H, bs), 6.90(1H, d, J=7.2Hz), 7.1-7.4(3H, m), 7.45(1H, s)

### (Compound number: Ia-23) 1-(5, 6-dimethyl pyridine-3-yl)-5-fluoro-3, 3, 4, 4-tetramethyl-3, 4-dihydroisoquinoline (physical property: amorphous)

¹H-NMR(300MHz, CDCl₃) δ ppm; 1.27(3H, bs), 1.41(3H, s), 1.42(3H, s), 2.33(3H, s), 2.55(3H, s), 6.95(1H, dd, J=1.8, 7.2Hz), 7.0-7.3(2H, m), 7.59(1H, d, J=1.8Hz), 8.39(1H, d, J=1.8Hz)

### (Compound number: Ia-58) 1-(5-methyl-6-trifluoromethyl pyridine-3-yl)-3, 3-dimethyl-5-fluoro-3, 4-dihydroisoquinoline (physical property: amorphous)

¹H-NMR(300MHz, CDCl₃) δ ppm; 1.38(6H, s), 2.50-2.60(3H, m), 2.85(2H, s), 6.95(1H, dd, J=8.0, 2.1Hz), 7.10-7.30(2H, m), 7.88(1H, s), 8.62(1H, s)

### (Example 2)

### Production of 1-(5-ethyl pyridine-3-yl)-3, 3-dimethyl-3, 4-dihydroisoquinoline (compound number: Ia-2)

0.5 g (1.59 mmol) of 1-(5-bromopyridine-3-yl)-3, 3-dimethyl-3, 4-dihydroisoquinoline (compound number: Ia-5) obtained in Example 1 and 0.04 g (0.08 mmol) of [1,2-bis(diphenyl phosphino)ethane] nickel(II) dichloride [NiCl₂(dppe)] were dissolved in 5 mL of dry THF. 0.7 mL (2.10 mmol) of ethyl magnesium bromide (3M diethyl ether solution) was dropped into the resulting solution at room temperature while stirring. After completion of the dropping, the reaction solution was stirred at room temperature for one night followed by pouring into ice water, and was adjusted to approximately pH 2 by a dilute hydrochloric acid followed by stirring at room temperature for 10 minutes. Next, the process liquid was neutralized by sodium carbonate and extracted with ethyl acetate followed by washing the organic layer with water and drying by addition of anhydrous magnesium sulfate. The resulting residue obtained by distilling off the solvent under reduced pressure was purified by silica gel column chromatography (developing solvent, n-hexane ethyl acetate = 3:1 (volume ratio)) to obtain 0.26 g (yield: 52%) of the target compound. The physical property was amorphous. ¹H-NMR data of the obtained compound are shown below.

¹H-NMR(300MHz, CDCl₃) δ ppm; 1.29(6H, s), 1.29(3H, t, J=7.5Hz), 2.71(2H, q, J=7.5Hz), 2.82(2H, s), 7.1-7.3(3H, m), 7.3-7.45(1H, m), 7.71(1H, m), 8.52(1H, d, J=2.1Hz), 8.59(1H, d, J=2.1Hz)

The compounds produced in the same manner as Example 2 are shown below. ¹H-NMR data of the obtained compounds are shown respectively.

### (Compound number: Ia-3) 1-(5-n-propyl pyridine-3-yl)-3, 3-dimethyl-3, 4-dihydroisoquinoline (physical property: amorphous)

¹H-NMR (300MHz, CDCl₃) δ ppm; 0.97(3H, t, J=7.5Hz), 1.29(6H, s), 1.69(2H, hep, J=7.5Hz), 2.64(2H, t, J=7.5Hz), 2.82(2H, s), 7.1-7.5(4H, m), 7.6-7.7(1H, m), 8.50(1H, d, J=2.4Hz), 8.60(1H, d, J=1.8Hz)

### (Compound number: Ia-8) 1-(6-ethyl pyridine-3-yl)-3, 3-dimethyl-3, 4-dihydroisoquinoline (physical property: amorphous) [0160]

¹H-NMR(300MHz, CDCl₃) δ ppm; 1.29(6H, s), 1.33(3H, t, J=7.8Hz), 2.82(2H, s), 2.88(2H, q, J=7.8Hz), 7.1-7.3(4H, m), 7.3-7.42(1H, m), 7.81(1H, dd, J=2.0, 8.0Hz), 8.69(1H, d, J=2.0Hz)

### (Compound number: Ia-9) 1-(6-n-propyl pyridine-3-yl)-3, 3-dimethyl-3, 4-dihydroisoquinoline (physical property: amorphous)

¹H-NMR(300MHz, CDCl₃) δ ppm; 0.99(3H, t, J=7.5Hz), 1.28(6H, s), 1.79(2H, hep, J=7.5Hz), 2.7-2.9(4H, m), 7.1-7.3(4H, m), 7.39(1H, dt, J=1.8, 8.0Hz), 7.81(1H, dd, J=2.4, 8.0Hz), 8.70(1H, d, J=2.4Hz)

### (Compound number: Ia-10) 1-(6-i-propyl pyridine-3-yl)-3, 3-dimethyl-3, 4-dihydroisoquinoline (physical property: amorphous)

¹H-NMR(300MHz, CDCl₃) δ ppm; 1.28(6H, s), 1.33(6H, d, J=6.5Hz), 2.81(2H, s), 3.12(1H, hep, J=6.5Hz), 7.1-7.3(4H, m), 7.3-7.4(1H, m), 7.82(1H, dd, J=2.0, 8.0Hz), 8.70(1H, d, J=2.0Hz)

### (Compound number: Ia-20) 1-(5-chloro-6-ethyl pyridine-3-yl)-3, 3-dimethyl-3, 4-dihydroisoquinoline (physical property: amorphous)

¹H-NMR(300MHz, CDCl₃)δppm; 1.27(6H, s), 1.32(3H, t, J=7.6Hz), 2.80(2H, s), 3.02(2H, q, J=7.6Hz), 7.1-7.5(4H, m), 7.88(1H, s), 8.58(1H, s)

### (Example 3)

### Production of 1-(5-vinyl pyridine-3-yl)-3, 3-dimethyl-3, 4-dihydroisoquinoline (compound number: Ia-4)

0.7 g (2.22 mol) of 1-(5-bromopyridine-3-yl)-3, 3-dimethyl-3, 4-dihydroisoquinoline (compound number: Ia-5) produced in Example 1 was dissolved in 20 mL of ethanol. Then, 0.34 g (3.37 mmol) of triethylamine, 0.33 g (2.46 mmol) of potassium vinyl trifluoroborate and 0.05 g (0.058 mmol) of [1,1'-bis(diphenyl phosphino)ferrocene] palladium(II)dichloride dichloromethane adductus [PdCl₂(dppf)-CH₂Cl₂] were added to the resulting solution in this order followed by heating the entire solution under reflux for 5 hours. The residue obtained by distilling off ethanol from the reaction solution under reduced pressure was dissolved in ethyl acetate and followed by washing with water and drying by addition of anhydrate magnesium sulfate. The residue was concentrated under reduced pressure and purified by silica gel column chromatography (developing solvent: n-hexane:ethyl acetate = 9:1 (volume ratio)) to obtain 0.48 g (yield: 82%) of the target compound. The physical property of the obtained compound was amorphous. ¹H-NMR data of the compound are shown below.

¹H-NMR(300MHz, CDCl₃) δ ppm; 1.30(6H, s), 2.83(2H, s), 5.42(1H, d, J=11.1Hz), 5.88(1H, d, J=17.4Hz), 6.75(1H, dd, J=11.1, 17.4Hz), 7.1-7.5(5H, m), 7.92(1H, m), 8.66(1H, dd, J=2.0, 8.4Hz)

The following compounds are produced in the same manner as Example 3. ¹H-NMR data of the produced compounds are shown below.

### (Compound number: Ia-12) 1-(6-vinyl pyridine-3-yl)-3, 3-dimethyl-3, 4-dihydroisoquinoline (physical property: amorphous)

¹H-NMR(300MHz, CDCl₃) δ ppm; 1.28(6H, s), 2.82(2H, s), 5.53(1H, d, J=10.5Hz), 6.26(1H, d, J=17.4Hz), 6.88(1H, dd, J=10.5, 17.4Hz), 7.1-7.5(5H, m), 7.89(1H, d, J=8.1 Hz), 8.75(1H, s)

### (Example 4)

### Production of 1-(6-cyanopyridine-3-yl)-3, 3-dimethyl-3, 4-dihydroisoquinoline (compound number: Ia-14)

0.5 g (1.85 mmol) of 1-(6-chloropyridine-3-yl)-3, 3-dimethyl-3, 4-dihydroisoquinoline (compound number: Ia-11), 0.21 g (0.19 mmol) of Pd(PPh₃)₄ and 0.22 g (1.85 mmol) of zinc cyanide were dissolved in 5 mL of DMF followed by stirring the entire solution for one night at 80°C. The reaction solution was poured into ice water and extracted with ethyl acetate. The organic layer was washed with water followed by drying by addition of anhydrous magnesium sulfate and distilling off the solvent under reduced pressure. The resulting residue was purified by silica gel column chromatography (developing solvent: n-hexane:ethyl acetate = 3:1 (volume ratio)) to obtain 0.06 g (yield: 14%) of the target compound. The physical property of the obtained compound was amorphous. ¹H-NMR data of the compound are shown below.

¹H-NMR(300MHz, CDCl₃) δ ppm; 1.29(6H, s), 2.83(2H, s), 7.16(1H, d, J=7.2Hz), 7.2-7.3(2H, m), 7.4-7.5(1H, m), 7.77(1H, d, J=1.8Hz), 8.06(1H, dd, J=2.1, 8.1Hz), 9.00(1H, d, J=2.1Hz)

### (Example 5)

### Production of 1-(6-phenyl pyridine-3-yl)-3,3-dimethyl-3, 4-dihydroisoquinoline (compound number: Ia-13)

0.5 g (1.85 mmol) of 1-(6-chloropyridine-3-yl)-3,3-dimethyl-3, 4-dihydroisoquinoline (compound number: Ia-11), 0.21 g (0.19 mmol) of Pd(PPh₃)₄, 0.34 g (2.78 mmol) of phenyl boronic acid(Ph-B(OH)₂) and 0.38 g (2.78 mmol) of potassium carbonate were dissolved in 10 mL of THF aqueous solution (50%) followed by heating the entire solution under reflux for one night. After cooling the reaction solution, the reaction solution was poured into ice water and extracted with ethyl acetate. The organic layer was washed with water and dried by addition of anhydrous magnesium sulfate followed by distilling off the solvent under reduced pressure. The resulting residue was purified by silica gel column chromatography (developing solvent: n-hexane:ethyl acetate = 3:1 (volumn ratio)) to obtain 0.35 g (yield: 61%) of the target compound. The physical property of the obtained compound was amorphous. ¹H-NMR data of the compound are shown below.

¹H-NMR(300MHz, CDCl₃) δ ppm; 1.28(6H, s), 2.83(2H, s), 7.2-7.5(7H, m), 7.77(1H, d, J=8.1Hz), 7.9-8.1(3H, m), 8.87(1H, d, J=1.8Hz)

### (Example 6)

### Production of 1-(5-chloro-6-methoxypyridine-3-yl)-3, 3-dimethyl-3, 4-dihydroisoquinoline (compound number: Ia-21)

0.60 g (1.96 mmol) of 1-(5, 6-dichloropyridine-3-yl)-3, 3-dimethyl-3, 4-dihydroisoquinoline (compound number: Ia-19) was dissolved in 10 mL of THF and 0.11 g (1.96 mmol) of sodium methoxide was added to the resulting solution followed by heating the entire solution under reflux for 5 hours. Then, a further 0.11 g of sodium methoxide was added to the solution followed by heating under reflux for one night. After cooling the reaction solution to room temperature, the reaction solution was poured into ice water and extracted with ethyl acetate. The organic layer was washed with water and dried by addition of anhydrous magnesium sulfate followed by distilling off the solvent under reduced pressure. The resulting residue was purified by silica gel column chromatography (developing solvent: n-hexane:ethyl acetate = 20:1 (volume ratio)) to obtain 0.30 g (yield: 51 %) of the target compound. The physical property of the obtained compound was amorphous. ¹H-NMR data of the compound are shown below.

¹H-NMR(300MHz, CDCl₃) δ ppm; 1.23(6H, s), 2.79(2H, s), 4.04(3H, s), 7.2-7.5(4H, m), 8.14(1H, d, J=1.8Hz), 8.25(1H, d, J=1.8Hz)

### (Example 7)

### Synthesis of 1-(5, 6-dimethyl pyridine-3-yl)-5-fluoro-3, 3, 4, 4-tetramethyl-1, 2, 3, 4-tetrahydroisoquinoline (compound number: Id-2)

0.33 g (1.06 mmol) of 1-(5, 6-dimethyl pyridine-3-yl)-5-fluoro-3, 3, 4, 4-tetramethyl-3, 4-dihydroisoquinoline (compound number: Ia-23) was dissolved in 20 mL of ethyl alcohol and 0.4 g (10.6 mol) of NaBH₄ was added to the resulting solution followed by heating the entire solution under reflux for 5 hours. Then, a further 0.2 g (5.3 mmol) of NaBH₄ was added to the reaction solution followed by heating under reflux for 5 more hours. After cooling the obtained reaction solution to room temperature, the reaction solution was poured into ice water and acidized (pH of 2 to 3) by 3N hydrochloric acid, and alkalified (pH of 9 to 10) by sodium carbonate followed by extraction with ethyl acetate. After washing the organic layer with water, the solution was dried by addition of anhydrous magnesium sulfate followed by distilling off the solvent under reduced pressure. The obtained residue was purified by silica gel column chromatography (developing solvent: chloroform:ethyl acetate = 95:5 (volume ratio)) to obtain 0.1 g (yield: 30%) of the target compound (physical property: amorphous).

¹H-NMR(300MHz, CDCl₃) δ ppm; 1.17(3H, s), 1.25(3H, s), 1.40-1.44(3H, m), 1.48(3H, s), 2.21(3H, s), 2.48(3H, s), 5.14(1H, s), 6.43(1H, d, J=8.0Hz), 6.85(1H, dd, J=8.0, 13.2Hz), 6.95(1H, dt, J=5.4, 8.0Hz), 7.22(1H, d, J=2.0Hz), 8.27(1H, d, J=2.0Hz)

The following compounds were produced in the same manner.

### (Compound number: Id-1) 3,3-dimethyl-1-(5, 6-dimethyl pyridine-3-yl-5-fluoro-1,2, 3, 4-tetrahydroisoquinoline (physical property: amorphous)

¹H-NMR(300MHz, CDCl₃) δ ppm; 1.22(3H, s), 1.32(3H, s), 2.22(3H, s), 2.49(3H, s), 2.66(1H, d, J=16.5Hz), 2.80(1H, d, J=16.5Hz), 5.10(1H, s), 6.49(1H, d, J=7.8Hz), 6.8-7.0(2H, m), 7.28(1H, d, J=1.8Hz), 8.30(1H, d, J=1.8Hz)

Although the following indicates some preparation examples of the fungicide of the present invention, the additives and addition ratios are not limited to those indicated in the examples and can be varied over a wide range. In addition, the term "parts" in the preparation examples indicates parts by weight.

### Preparation Example 1 Wettable Powder

| | |
|---|---|
| Compound of the present invention | 40 parts |
| Clay | 48 parts |
| Sodium dioctylsulfosuccinate | 4 parts |
| Sodium lignin sulfonate | 8 parts |

The above components are uniformly mixed and finely crushed to obtain a wettable powder containing 40% of the active ingredient.

### Preparation Example 2 Emulsion

| | |
|---|---|
| Compound of the present invention | 10 parts |
| Solvesso 200 | 53 parts |
| Cylohexanone | 26 parts |
| Calcium dodecylbenzenesulfonate | 1 part |
| Polyoxyethylene alkyl allyl ether | 10 parts |

The above components are mixed and dissolved to obtain an emulsion containing 10% of the active ingredient.

### Preparation Example 3 Powder

| | |
|---|---|
| Compound of the present invention | 10 parts |
| Clay | 90 parts |

The above components are uniformly mixed and finely crushed to obtain a powder containing 10% of the active ingredient.

### Preparation Example 4 Granules

| | |
|---|---|
| Compound of the present invention | 5 parts |
| Clay | 73 parts |
| Bentonite | 20 parts |
| Sodium dioctylsulfosuccinate | 1 part |
| Potassium phosphate | 1 part |

The above components are finely crushed and mixed well followed by the addition of water, mixing well, granulating and drying to obtain granules containing 5% of the active ingredient.

### Preparation Example 5 Suspension

| | |
|---|---|
| Compound of the present invention | 10 parts |
| Polyoxyethylene alkyl allyl ether | 4 parts |
| Sodium polycarbonate | 2 parts |
| Glycerin | 10 parts |
| Xanthan gum | 0.2 parts |
| Water | 73.8 parts |

The above components are mixed followed by wet-crushing to a particle diameter of 3 microns or less to obtain a suspension containing 10% of the active ingredient.

### Preparation Example 6 Water Dispersible Granules

| | |
|---|---|
| Compound of the present invention | 40 parts |
| Clay | 36 parts |
| Potassium chloride | 10 parts |
| Sodium alkylbenzenesulfonate | 1 part |
| Sodium lignin sulfonate | 8 parts |
| Formaldehyde condensation product of sodium alkylbenzenesulfonate | 5 parts |

The above components are uniformly mixed and finely crushed followed by adding a suitable amount of water and mixing to form a clay-like mixture. The clay-like mixture is granulated and dried to obtain water dispersible granules containing 40% of the active ingredient.

### (Test Example 1) Apple Scab Control Test

Emulsions of fungicide of the present invention were sprayed at an active ingredient concentration of 100 ppm onto apple seedlings (variety: Ralls Janet, leaf stage: 3 to 4) cultivated in unglazed pots. After allowing to air-dry at room temperature, the seedlings were inoculated with conidiospores of apple scab pathogen (Venturia inaequalis) followed by holding for 2 weeks indoors at 20°C and high humidity using a 12 hour light/dark cycle. The appearance of lesions on the leaves was compared with untreated seedlings to determine control effects.

As a result, the following compounds demonstrated superior control values of 75% or more.

### Compound numbers (compound numbers correspond to compound numbers listed in the above-mentioned tables): Ia-12, Ia-15, Ia-16, Ia-17, Ia-18, Ia-19, Ia-20, Ia-23, Id-1, Id-2

### INDUSTRIAL APPLICABILITY

The nitrogen-containing heterocyclic compound and the salt thereof of the present invention are new compounds and they can be produced industrially advantageously, they can be used as an active ingredient of a fungicide for agricultural and horticultural use which is definitely effective and safe to use.

Further, the fungicide for agricultural and horticultural use of the present invention has excellent control effect, does not cause drug disaster or contamination to plants, and demonstrates less toxicity for humans, livestock or marine life and has less environmental impact. Therefore, the present invention has industrial benefits.

## Claims

1. A nitrogen-containing heterocyclic compound represented by the formula (I) 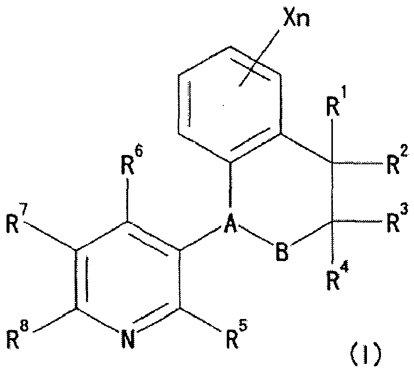 (wherein, A-B represents formula II or III; 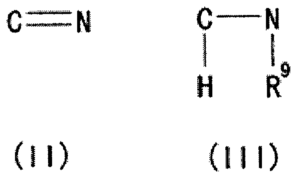
R¹, R², R³ and R⁴ independently represent hydrogen atom, a C1-20 alkyl group, provided that when A-B represents the formula (II) or (III), R¹, R², R³ and R⁴ do not all represent hydrogen atom;
R⁵ and R⁶ represent a hydrogen atom.
R⁷ and R⁸ independently represent hydrogen atom, a halogen atom, a C1-20 alkyl group, a C1-20 haloalkyl group, a C2-20 alkenyl group, a C2-20 haloalkenyl group, a C2-20 alkynyl group, a C2-20 haloalkynyl group, a C1-20 alkoxy group, a C1-20 haloalkoxy group, a C3-20 cycloalkyl group, a C3-20 cycloalkoxy group, a C2-20 alkenyloxy group, a C2-20 alkynyloxy group, a C1-20 alkyl thio group, cyano group, an unsubstituted or substituted amino group, nitro group, an unsubstituted or substituted phenyl group, an unsubstituted or substituted aralkyl group, an unsubstituted or substituted aryloxy group, an unsubstituted or substituted heterocyclic group, or an unsubstituted or substituted heteroaryloxy group, provided that when A-B represents the formula (II) or (III), R⁷ and R⁸ do not both represent hydrogen atom, and when A-B represents the formula (III) and R⁷ represents hydrogen atom, R⁸ does not represent methyl group;
X represents a halogen atom;
n represents an integer of 0 to 4;
in the formula (III), R⁹ represents hydrogen atom,
and a salt thereof.

2. A fungicide for agricultural and horticultural use, comprising at least one of the compound and the salt thereof according to claim 1.

## Patentansprüche

1. Stickstoffhaltige heterocyclische Verbindung, die durch die Formel (I) dargestellt wird 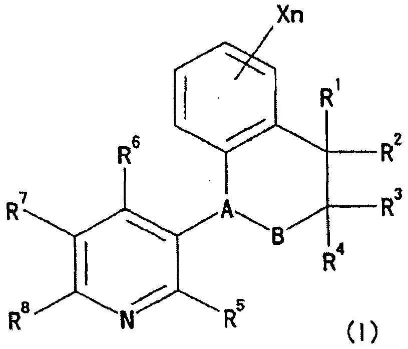 (in der A-B die Formel II oder III darstellt; 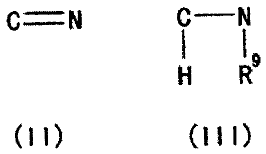
R¹, R², R³ und R⁴ unabhängig ein Wasserstoffatom, eine (C1-20)-Alkylgruppe darstellen, vorausgesetzt, dass, wenn A-B die Formel (II) oder (III) darstellt, R¹, R², R³ und R⁴ nicht alle ein Wasserstoffatom darstellen;
R⁵ und R⁶ ein Wasserstoffatom darstellen;
R⁷ und R⁸ unabhängig ein Wasserstoffatom, ein Halogenatom, eine (C1-20)-Alkylgruppe, eine (C1-20)-Halogenalkylgruppe, eine (C2-20)-Alkenylgruppe, eine (C2-20)-Halogenalkenylgruppe, eine (C2-20)-Alkinylgruppe, eine (C2-20)-Halogenalkinylgruppe, eine (C1-20)-Alkoxygruppe, eine (C1-20)-Halogenalkoxygruppe, eine (C3-20)-Cycloalkylgruppe, eine (C3-20)-Cycloalkoxygruppe, eine (C2-20)-Alkenyloxygruppe, eine (C2-20)-Alkinyloxygruppe, eine (C1-20)-Alkylthiogruppe, eine Cyanogruppe, eine unsubstituierte oder substituierte Aminogruppe, eine Nitrogruppe, eine unsubstituierte oder substituierte Phenylgruppe, eine unsubstituierte oder substituierte Aralkylgruppe, eine unsubstituierte oder substituierte Aryloxygruppe, eine unsubstituierte oder substituierte heterocyclische Gruppe oder eine unsubstituierte oder substituierte Heteroaryloxygruppe darstellen, vorausgesetzt, dass, wenn A-B die Formel (II) oder (III) darstellt, R⁷ und R⁸ nicht beide ein Wasserstoffatom darstellen, und wenn A-B die Formel (III) darstellt und R⁷ ein Wasserstoffatom darstellt, R⁸ keine Methylgruppe darstellt;
X ein Halogenatom darstellt;
n eine ganze Zahl von 0 bis 4 darstellt;
in der Formel (III) R⁹ ein Wasserstoffatom darstellt,
und ein Salz derselben.

2. Ein Fungizid für die landwirtschaftliche oder gartenbauliche Verwendung, umfassend mindestens eines aus der Verbindung und dem Salz derselben nach Anspruch 1.

## Revendications

1. Composé hétérocyclique contenant un atome d'azote représenté par la formule (I) 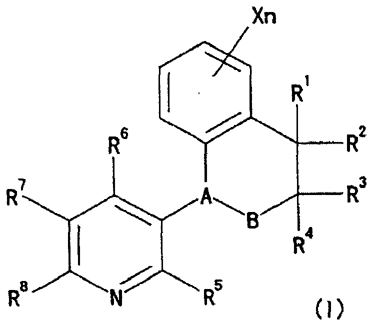 (dans laquelle, A-B représente la formule II ou III ; 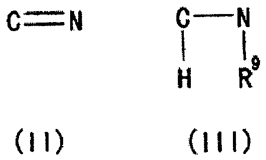
R¹, R², R³ et R⁴ représentent indépendamment un atome d'hydrogène, un groupe alkyle en C1-20, à condition que lorsque A-B représente la formule (II) ou (III), R¹, R², R³ et R⁴ ne représentent pas tous un atome d'hydrogène ;
R⁵ et R⁶ représentent un atome d'hydrogène.
R⁷ et R⁸ représentent indépendamment un atome d'hydrogène, un atome d'halogène, un groupe alkyle en C1-20, un groupe halogénoalkyle en C1-20, un groupe alcényle en C2-20, un groupe halogénoalcényle en C2-20, un groupe alcynyle en C2-20, un groupe halogénoalcynyle en C2-20, un groupe alcoxy en C1-20, un groupe halogénoalcoxy en C1-20, un groupe cycloalkyle en C3-20, un groupe cycloalcoxy en C3-20, un groupe alcényloxy en C2-20, un groupe alcynyloxy en C2-20, un groupe alkylthio en C1-20, un groupe cyano, un groupe amino non substitué ou substitué, un groupe nitro, un groupe phényle non substitué ou substitué, un groupe aralkyle non substitué ou substitué, un groupe aryloxy non substitué ou substitué, un groupe hétérocyclique non substitué ou substitué, ou un groupe hétéroaryloxy non substitué ou substitué, à condition que lorsque A-B représente la formule (II) ou (III), R⁷ et R⁸ ne représentent pas tous deux un atome d'hydrogène, et lorsque A-B représente la formule (III) et R⁷ représente un atome d'hydrogène, R⁸ ne représente pas un groupe méthyle ;
X représente un atome d'halogène ;
n représente un nombre entier de 0 à 4 ;
dans la formule (III), R⁹ représente un atome d'hydrogène,
et un sel de celui-ci.

2. Fongicide pour une utilisation en agriculture et en horticulture, comprenant au moins un parmi le composé et le sel de celui-ci selon la revendication 1.
